(19) Europäisches Patentamt / European Patent Office / Office européen des brevets

(11) **EP 3 105 573 B1**

(12) **FASCICULE DE BREVET EUROPEEN**

(45) Date de publication et mention de la délivrance du brevet:
**28.02.2024 Bulletin 2024/09**

(21) Numéro de dépôt: **15706908.9**

(22) Date de dépôt: **12.02.2015**

(51) Classification Internationale des Brevets (IPC):
**G01N 27/24** *(2006.01)*  **G01N 33/44** *(2006.01)*

(52) Classification Coopérative des Brevets (CPC):
**G01N 27/24; G01N 33/442;** G01N 33/44

(86) Numéro de dépôt international:
**PCT/FR2015/050347**

(87) Numéro de publication internationale:
**WO 2015/121591 (20.08.2015 Gazette 2015/33)**

(54) **PROCÉDÉ DE DÉTECTION DE DÉFAUT DE SOUDURE**

VERFAHREN ZUR ERKENNUNG VON DEFEKTEN SCHWEISSNÄHTEN

METHOD FOR DETECTING WELD DEFECTS

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **12.02.2014 FR 1451074**

(43) Date de publication de la demande:
**21.12.2016 Bulletin 2016/51**

(73) Titulaire: **Electricité de France**
**75008 Paris (FR)**

(72) Inventeur: **TAILLADE, Frédéric**
**92140 Clamart (FR)**

(74) Mandataire: **Plasseraud IP**
**66, rue de la Chaussée d'Antin**
**75440 Paris Cedex 09 (FR)**

(56) Documents cités:
EP-A1- 1 061 361   EP-A2- 1 267 158
DE-A1- 3 504 609   US-A1- 2009 261 846
US-B1- 7 839 282

- MATHUR M ET AL: "A probe for in situ , remote, detection of defects in buried plastic natural gas pipelines", REVIEW OF SCIENTIFIC INSTRUMENTS, AIP, MELVILLE, NY, US, vol. 78, no. 12, 18 décembre 2007 (2007-12-18), pages 125105-125105, XP012105579, ISSN: 0034-6748, DOI: 10.1063/1.2821238
- BOWMAN J: "A REVIEW OF THE ELECTROFUSION JOINING PROCESS FOR POLYETHYLENE PIPE SYSTEMS", POLYMER ENGINEERING AND SCIENCE, BROOKFIELD CENTER, US, vol. 37, no. 4, 1 avril 1997 (1997-04-01), pages 674-691, XP000697916, ISSN: 0032-3888, DOI: 10.1002/PEN.11712
- JIANFENG SHI ET AL: "Defects classification and failure modes of electrofusion joint for connecting polyethylene pipes", JOURNAL OF APPLIED POLYMER SCIENCE, vol. 124, no. 5, 5 juin 2012 (2012-06-05), pages 4070-4080, XP055123214, ISSN: 0021-8995, DOI: 10.1002/app.35013
- KAFIEH R ET AL: "Automatic detection of defects on polyethylene pipe welding using thermal infrared imaging", INFRARED PHYSICS AND TECHNOLOGY, vol. 54, no. 4, 25 décembre 2010 (2010-12-25), pages 317-325, XP028387785, ISSN: 1350-4495, DOI: 10.1016/J.INFRARED.2010.12.010 [extrait le 2010-12-25]
- ZHU XINWEI ET AL: "Identification of defects in HDPE piping thermal fusion welds based on microwave inspection and true density measurement", 2013 FAR EAST FORUM ON NONDESTRUCTIVE EVALUATION/TESTING: NEW TECHNOLOGY AND APPLICATION, IEEE, 17 juin 2013 (2013-06-17), pages 212-215, XP032503660, DOI: 10.1109/FENDT.2013.6635559 ISBN: 978-1-4673-6018-0 [extrait le 2013-10-16]

- **Agilent Technologies: "Agilent 4291B RF Impedance/Material Analyzer", , 13 July 2006 (2006-07-13), XP055787524, Retrieved from the Internet: URL:https://www.keysight.com/dk/en/assets/ 7018-09069/data-sheets-archived/5966-1543. pdf [retrieved on 2021-03-18]**

**Description**

La présente invention concerne un procédé de détection d'un défaut dans une soudure faisant jonction entre une première surface d'un matériau à base de polyéthylène et une deuxième surface d'un matériau à base de polyéthylène.

Elle trouve une application dans de nombreux domaines, tels que ceux des centrales nucléaires ou hydrauliques, et, plus généralement, dans toute industrie où l'on a recours à des conduits en matériau à base de polyéthylène (PE), généralement de haute densité (PEHD), comme des réseaux d'eau potable, de gaz naturel, ou encore sur certaines lignes électriques.

En effet, il est connu de remplacer des canalisations existantes, notamment en acier, par des conduits en PE, qui permettent d'éviter des problèmes de corrosion et d'entretien des canalisations en acier, de renforcer la résistance aux séismes des installations, tout en assurant une réduction du coût et du délai de changement des conduits.

Un réseau de conduits en PE est obtenu par soudage des conduits de PE par leurs extrémités, où une soudure fait jonction entre une première surface d'un matériau à base de PE, par exemple une extrémité d'un premier conduit en PE, et une deuxième surface d'un matériau à base de PE, par exemple une extrémité d'un second conduit en PE.

La qualité de la soudure est par conséquent primordiale pour une utilisation sûre et pérenne d'un tel réseau. Cependant, selon les conditions dans lesquelles est opéré le soudage, il est possible que la soudure soit contaminée par des impuretés comme de la graisse, des poussières, ou encore que soit localement créée une poche d'air par manque de fusion, ce qui génère un autre type de défaut, dit défaut de fusion froide.

La qualité de la soudure peut également être altérée par son vieillissement.

Or, il peut être essentiel de contrôler la qualité de la soudure, pour s'assurer du bon fonctionnement des réseaux de conduits en PE. Il est donc besoin d'un procédé de détection d'un défaut d'une telle soudure.

Un procédé connu se base sur l'utilisation d'ondes ultrasonores. Néanmoins, ce procédé est limité à la détection de défauts macroscopiques, de dimension de l'ordre de plusieurs millimètres carrés, mais ne permet pas de déceler des défauts plus petits, et, en particulier, ne permet pas de déceler les défauts de fusion froide.

L'article "A probe for in situ, remote, détection of defects in buried plastic natural gas pipelines", Mathur M et al, Review of Scientific Instruments, vol. 78, no. 12, 18 décembre 2007, ainsi que le brevet US7839282B1 décrivent la détection de défauts dans des tubes en polyéthylène au moyen d'une sonde capacitive.

Le but de la présente invention est de remédier à ces inconvénients.

A cet effet, l'invention a pour objet un procédé de détection d'un défaut dans une soudure faisant jonction entre une première surface d'un matériau à base de polyéthylène et une deuxième surface d'un matériau à base de polyéthylène selon la revendication indépendante 1, ledit procédé comprenant les étapes :

- positionnement d'une première électrode et d'une deuxième électrode à proximité de la soudure, de sorte qu'au moins une ligne de champ électrique traverse ladite soudure quand une différence de potentiel est appliquée entre lesdites première et deuxième électrodes,
- mesure d'une capacité électrique à une fréquence de mesure entre ladite première électrode et ladite deuxième électrode, la fréquence de mesure étant supérieure à 65 MHz et inférieure à 1 GHz pour comparaison de la capacité électrique mesurée à une valeur de référence.

Ainsi, le procédé selon la présente invention permet de détecter non seulement des défauts de taille macroscopique, de l'ordre de quelques millimètres carrés, mais aussi des défauts de taille d'une dizaine de nanomètres et des défauts de type fusion froide.

Dans une réalisation particulière, la fréquence de mesure est mesurée entre 100MHz et 300MHz.

Sur cette plage fréquentielle de fonctionnement l'impédance électrique mesurée entre les deux électrodes est assimilable en première approximation à une capacité électrique.

Dans une réalisation particulière, le procédé de détection comprend une étape de détection d'un défaut dans la soudure si, lors de l'étape de comparaison, la capacité électrique mesurée est différente d'au moins 1,5% de la valeur de référence Cref.

Dans une réalisation particulière, la valeur de référence est de l'ordre de 6pF.

Dans une réalisation particulière, la première surface appartient à un corps présentant une épaisseur et la deuxième surface appartient à un corps présentant une épaisseur du même ordre de grandeur que l'épaisseur du corps de la première surface, et la distance prédéfinie entre la première électrode et la deuxième électrode est comprise dans une plage de valeurs entre le cinquième de la valeur de ladite épaisseur et ladite épaisseur.

Cette plage de valeur assure une capacité électrique mesurée suffisamment différente entre une soudure avec défaut et une soudure sans défaut pour que le défaut soit détecté par le procédé.

Une sonde capacitive pour la mise en oeuvre du procédé tel que décrit précédemment, cette sonde ne faisant pas partie de l'invention, comprend une première électrode et une deuxième électrode formant condensateur présentant une capacité électrique apte à dépendre de la présence d'un défaut dans une soudure faisant jonction entre une première surface d'un matériau à base de polyéthylène et une deuxième surface d'un matériau à base de polyéthylène, et un dispositif relié audit condensateur apte à mesurer la valeur de capacité dans une fréquence de mesure entre 65 MHz et 1 GHz.

Une telle sonde est adaptée au procédé de détection précédemment décrit.

Par exemple, le dispositif est un circuit oscillant dont la mesure de la fréquence de résonance permet de déduire la capacité électrique mesurée ou un montage en pont ou un impédance mètre ou un analyseur de réseau vectoriel.

Par exemple, la fréquence de mesure est comprise entre 100MHz et 300MHz.

Par exemple, la première électrode et la deuxième électrode sont d'une longueur de l'ordre d'une épaisseur d'un corps comprenant ladite première surface et d'un corps comprenant ladite deuxième surface.

Par exemple, la première électrode est constituée d'un bobinage de fils électriques.

Selon la revendication indépendante 6, l'invention a également pour objet une utilisation d'une sonde capacitive pour la détection de défaut dans une soudure faisant jonction entre une première surface d'un matériau à base de polyéthylène et une deuxième surface d'un matériau à base de polyéthylène.

Dans une réalisation particulière, la première surface est coaxiale et adjacente de la deuxième surface.

Dans une réalisation particulière, la première surface est coaxiale et emmanche en partie au moins la deuxième surface.

D'autres caractéristiques et avantages de l'invention apparaîtront encore à la lecture de la description qui va suivre. Celle-ci est purement illustrative et doit être lue en regard des dessins annexés sur lesquels :

- La figure 1 est une vue en coupe longitudinale d'un détail d'une installation de conduits en PEHD (polyéthylène haute densité) à laquelle est appliquée un procédé de détection de défaut selon la présente invention et sur lequel sont positionnés une première et une deuxième électrodes d'une sonde capacitive selon la présente invention ;

- La figure 2 est un ordinogramme du procédé de détection de défaut selon la présente invention ;

- La figure 3 est un schéma de l'évolution d'une capacité électrique mesurée dans une plage fréquentielle de fonctionnement ;

- La figure 4 est une vue du détail de la figure 1 illustrant la sensibilité de la sonde capacitive de la figure 2 en fonction de la distance entre les deux électrodes ;

- La figure 5 est un schéma de la valeur de la capacité électrique mesurée dans la plage fréquentielle de fonctionnement préférentiel ;

- La figure 6 est un schéma de la différence relative de la capacité électrique mesurée de la figure 5 ;

- La figure 7 est une vue en coupe longitudinale d'un détail d'une autre installation de conduits en PEHD à laquelle est appliqué le procédé de détection de défaut selon la présente invention ; et

- La figure 8 est une variante de la figure 7.

L'invention a pour objet un procédé de détection d'un défaut dans une soudure faisant jonction entre une première surface d'un matériau à base de polyéthylène (PE) et une deuxième surface d'un matériau à base de polyéthylène (PE).

Le procédé de détection selon la présente invention est présenté ci-après dans son application à une installation de conduits en polyéthylène haute densité (PEHD), cette application n'étant bien entendue pas limitative de la portée de l'invention.

Mode de réalisation du procédé de détection de défaut à une installation de conduits en PEHD

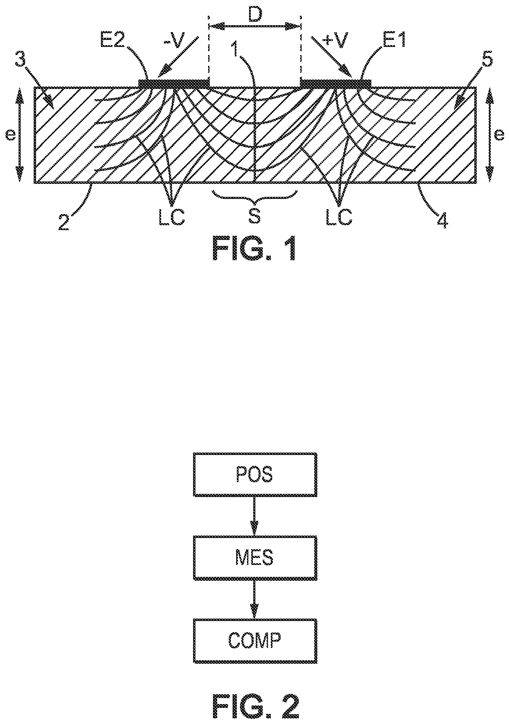

Sur la figure 1 est illustré un détail d'une installation de conduits en PEHD, une soudure S faisant jonction 1 entre deux conduits, la première surface étant une surface de pourtour 2 d'un premier conduit 3 et la deuxième surface étant une surface de pourtour 4 d'un deuxième conduit 5.

La première surface 2 et la deuxième surface 4 présentent une forme générale de cylindres coaxiaux d'une épaisseur e.

Comme visible sur la figure 1, la soudure S est déposée entre deux extrémités adjacentes des conduits 3 et 5.

Comme visible sur la figure 1, une première électrode E1 est disposée sur le côté extérieur de la surface de pourtour 2 du premier conduit 3 et une deuxième électrode E2 est disposée sur le côté extérieur de la surface de pourtour 4 du deuxième conduit 5.

Les électrodes E1 et E2 sont réalisées en matériau conducteur électriquement, par exemple un métal, tel que l'inox. Elles sont de préférence de forme et de dimensions identiques.

L'électrode E1 est au potentiel +V, tandis que l'électrode E2 est au potentiel-V.

Du fait de la différence de potentiel entre les électrodes E1 et E2, des lignes de champ électrique LC sont distribuées entre les deux électrodes, comme représentées à la figure 1 dont certaines passent dans la soudure S.

Sur les figures, les électrodes sont représentées au contact des surfaces externes 2 et 4 des conduits 3 et 5. Toutefois, l'invention n'est pas limitée à ce mode de réalisation.

Il est possible que les électrodes E1 et E2 soient disposées au contact des surfaces internes des conduits 3 et 5.

Il est également possible que les électrodes ne soient pas en contact avec les conduits 3 et 5 mais au contraire disposées à une distance non nulle des surfaces des conduits 3 et 5, soit à l'intérieur de ceux-ci, si les conditions le permettent, soit à l'extérieur de ceux-ci.

Selon l'invention, les électrodes E1 et E2 doivent être disposées à proximité de la soudure S, c'est-à-dire que les électrodes E1 et E2 sont disposées de sorte qu'au moins une ligne de champ LC traverse la soudure S.

La distance entre les électrodes E1 et E2 est notée D, et est choisie dans une plage de valeurs, comme il sera indiqué plus loin.

Procédé de détection

Le procédé de détection comprend les étapes :

- positionnement des électrodes E1 et E2 à proximité de la soudure S, comme expliqué ci-dessus. Cette étape est référencée POS à la figure 2, et

- mesure d'une capacité électrique Cmes à une fréquence de mesure entre la première électrode E1 et la deuxième électrode E2, la fréquence de mesure étant supérieure à 65 MHz et inférieure à 1 GHz pour comparaison de la capacité électrique mesurée à une valeur de référence Cref. L'étape de mesure est référencée MES sur la figure 2 et l'étape de comparaison COMP.

La capacité électrique mesurée Cmes dépend de la permittivité des matériaux traversés par les lignes de champ, qui dépend elle-même de la fréquence de mesure. En particulier, la capacité électrique dépend de la permittivité du matériau à base de PEHD dont sont constitués les conduits 3 et 5, la permittivité du matériau dont est constituée la soudure S, et éventuellement de la permittivité de l'air si les électrodes E1 et E2 ne sont pas directement au contact des surfaces 2 et 4.

Ainsi, un défaut dans la soudure S induit un changement local dans les matériaux traversés par les lignes de champ LC entre les deux électrodes E1 et E2, ce qui modifie la permittivité locale et par conséquent, la capacité électrique mesurée.

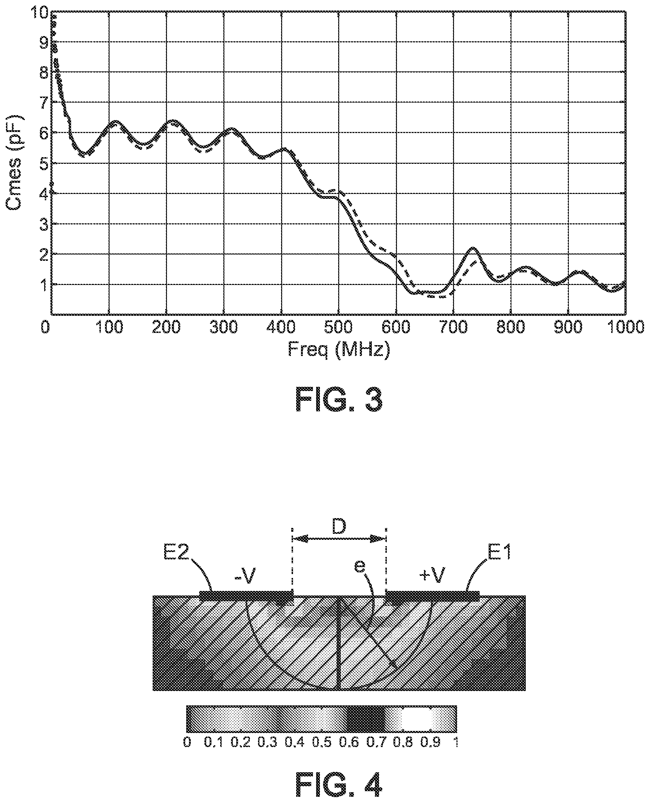

Comme illustré à la figure 3, la capacité électrique Cmes est mesurée pour une fréquence de mesure entre 0 et 1GHz, pour une jonction bien soudée (ligne discontinue) et pour une jonction avec défaut S de soudure (ligne continue). On observe que la capacité électrique mesurée Cmes est d'une valeur différente pour la jonction bien soudée et la jonction mal soudée, ce qui permet au procédé de détecter le défaut S.

Préférentiellement, la fréquence de mesure est mesurée entre 100MHz et 300MHz. Sur cette plage fréquentielle de fonctionnement l'impédance électrique mesurée entre les deux électrodes est assimilable en première approximation à une capacité.

Et, dans cette plage de valeurs, la différence de capacité entre une jonction bien soudée et une jonction présentant un défaut de soudure est particulièrement détectable, comme visible sur la figure 3.

Si la capacité électrique mesurée Cmes est différente d'au moins 1,5% à la valeur de référence Cref, un défaut est détecté dans la soudure S.

La valeur de référence est de préférence de l'ordre de 5 à 10pF, de préférence de 5 à 7 pF, avantageusement de l'ordre de 6pF, comme visible sur la figure 3, pour une fréquence de mesure comprise entre 100 et 300 MHz.

Dans ce cas, le procédé détecte un défaut dans la soudure si, lors de l'étape de comparaison, la capacité électrique mesurée est différente d'au moins 0,10 pF à la valeur de référence de 6 pF, et de préférence différente d'au moins 0,15 pF à la valeur de référence de 6 pF.

Alternativement, le procédé détecte un défaut dans la soudure S si la différence relative entre la capacité mesurée Cmes et la valeur de référence Cref, définie par $|Cmes - Cref|/Cref$ est supérieure à une incertitude de mesure de la capacité électrique.

De préférence, le procédé comprend une étape d'étalonnage de la mesure de la capacité électrique. En effet, la capacité électrique mesurée dépend de nombreux paramètres, tels que la géométrie des électrodes E1 et E2, de la distance entre E1 et E2, l'épaisseur e des conduits, la nature du matériau des conduits, et la fréquence de mesure, comme il sera détaillé plus loin. Ainsi, l'étape d'étalonnage permet de ne faire dépendre la capacité de mesure Cmes que de la qualité de soudure de la jonction.

Cette étape peut être expérimentale en réalisant une mesure dans l'air ou en utilisant des matériaux de références.

Sonde capacitive

Les électrodes E1 et E2 sont reliées à un dispositif de mesure apte à mesurer une capacité électrique dans la plage de fréquence entre 0 et 1GHz, et particulièrement entre 100MHz et 300MHz.

Ce dispositif pourra être par exemple un circuit oscillant dont la mesure de la fréquence de résonance permet de déduire la capacité électrique mesurée Cmes ; un montage en pont (Wheatstone, Nernst, Sauty, etc.) ; un impédance mètre ; un analyseur de réseau vectoriel, etc.

Si par exemple, le dispositif est un circuit oscillant, les électrodes E1 et E2 sont reliées à celui-ci. La mesure de la fréquence de résonance fosc permet de déduire la capacité électrique mesurée Cmes.

Les électrodes E1 et E2 et le dispositif de mesure de capacité électrique constituent une sonde capacitive.

Les électrodes E1 et E2 forment un condensateur dont la capacité électrique est la capacité électrique mesurée Cmes.

La fréquence de résonance est déterminée par la formule $fosc = \dfrac{1}{2\pi\sqrt{LCmes}}$, où L est l'inductance du circuit oscillant. La capacité électrique mesurée Cmes est déterminée par la formule $Cmes = \varepsilon0\varepsilon\chi$, où $\varepsilon0$ est la permittivité absolue dans le vide, qui est de l'ordre de $8,85.10^{-12}$F/m, $\varepsilon$ est la permittivité relative des matériaux traversés par les lignes de champ LC et le paramètre $\chi$ est un facteur dépendant de la géométrie des électrodes ($\chi = s/D$, où s est la surface des électrodes E1 et E2 et D est la distance entre les électrodes E1 et E2, dans un modèle de condensateur droit).

Comme il ressort des formules précédentes, la capacité mesurée Cmes dépend des caractéristiques de la géométrie de la sonde électrique, en particulier de la surface des électrodes E1 et E2, et de la distance D entre les électrodes E1 et E2.

La capacité électrique mesurée dépend également de la permittivité électrique $\varepsilon$ des matériaux traversés, comme déjà expliqué, cette permittivité dépendant de la fréquence de mesure.

La figure 4 illustre une sensibilité relative de la capacité mesurée Cmes par rapport à la position spatiale d'une variation de permittivité électrique (pouvant être due à la présence d'un défaut) selon la présente invention en fonction de la distance D prédéfinie entre les électrodes E1 et E2. On observe que la sonde peut être utilisée dans le procédé de détection de défaut si la distance D est inférieure ou de l'ordre de l'épaisseur e des conduits 3 et 5, de préférence comprise dans une plage de valeur entre e/5 et e.

On note également que la valeur de la capacité électrique mesurée Cmes (le signal) augmente avec la longueur des électrodes tandis que l'incertitude de mesure (le bruit) de la capacité électrique mesurée diminue avec la longueur des électrodes.

Le rapport signal sur bruit augmente avec la longueur des électrodes, tandis que la localisation spatiale d'un défaut S diminue avec cette longueur. Un bon compromis entre ces deux tendances contraires est évalué à une longueur pour les électrodes E1 et E2 de l'ordre de l'épaisseur e des conduits 3 et 5, de préférence de l'ordre de 50 mm, par exemple entre 45 mm et 55 mm. La géométrie des électrodes pourra être ajustée en fonction de la taille minimum des défauts recherchés.

Résultat expérimental

Dans l'expérience réalisée, les électrodes E1 et E2 sont constituées chacune d'un ruban adhésif de forme générale sensiblement rectangulaire. Leur longueur est de l'ordre de 20mm et leur largeur de l'ordre de 10 mm.

L'électrode E1 est disposée sur la surface externe 2 du premier conduit 3, la direction de la longueur correspondant à la direction axiale des conduits 3 et 5, tandis que la largeur correspond à la direction radiale des conduits 3 et 5.

Les électrodes E1 et E2 sont positionnées à une distance D de l'ordre de 22mm.

Les électrodes E1 et E2 sont reliées à un câble coaxial de type RG58 et un dispositif analyseur de réseau vectoriel (de type VNA, commercialisé sous la référence Anritsu 2026C) permet de réaliser une mesure en réflexion d'un coefficient S11 sur la plage fréquentielle de fonctionnement préférentiel, c'est-à-dire entre 100MHz et 300MHz.

Le coefficient S11 est déterminé par la formule suivante, $S11 = \dfrac{z-z0}{z+z0}$ où z0 est l'impédance du câble coaxial, de l'ordre de 50$\Omega$, tandis que l'impédance z est sensiblement égale à $z = -j/Cmes\omega$, j étant le nombre complexe $\sqrt{-1}$, $\omega$ étant la pulsation, $\omega = 2\pi f$ ; f est la fréquence de mesure et Cmes la capacité électrique mesurée.

La capacité électrique Cmes est alors mesurée en fonction de la fréquence de mesure, entre 100MHz et 300MHz.

Dans un premier temps, le procédé est appliqué à une installation dans laquelle la soudure S est de bonne qualité.

Puis, la même expérience est réitérée sur une installation dans laquelle la soudure S est défectueuse.

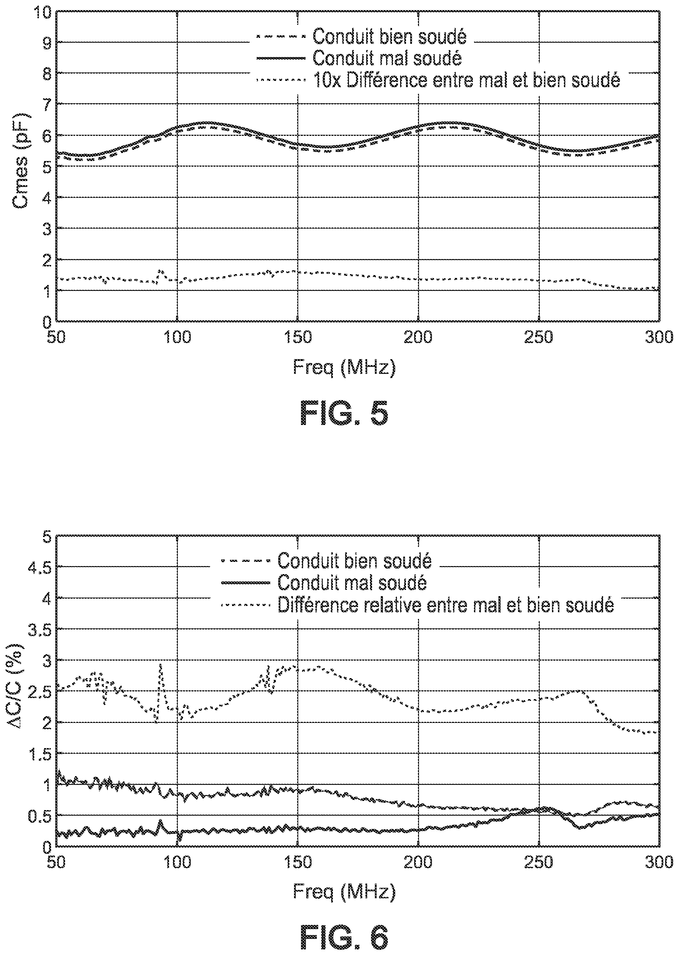

Les résultats expérimentaux obtenus sont illustrés aux figures 5 et 6.

Comme il ressort de la figure 5, la capacité mesurée Cmes est de l'ordre de 6 pF entre 50MHz et 300MHz.

La différence de capacité mesurée entre l'installation avec une bonne soudure et une soudure avec défaut est de l'ordre de 0,15 pF, ce qui représente une différence relative de capacité de l'ordre de 2,5%.

La figure 6 illustre l'évolution de la valeur de la différence relative de capacité en fonction de la fréquence de mesure, pour une fréquence de mesure entre 50 et 300 MHz.

L'incertitude de mesure de la capacité électrique dans cette expérience est évaluée à 1,5% par mesures de répétabilité de l'expérience.

Ainsi, la différence relative de capacité étant supérieure à l'incertitude de mesure, le défaut de soudure est détecté par le procédé selon la présente invention.

Mode de réalisation du procédé de détection de défaut à une installation de conduits en PEHD avec bobinage

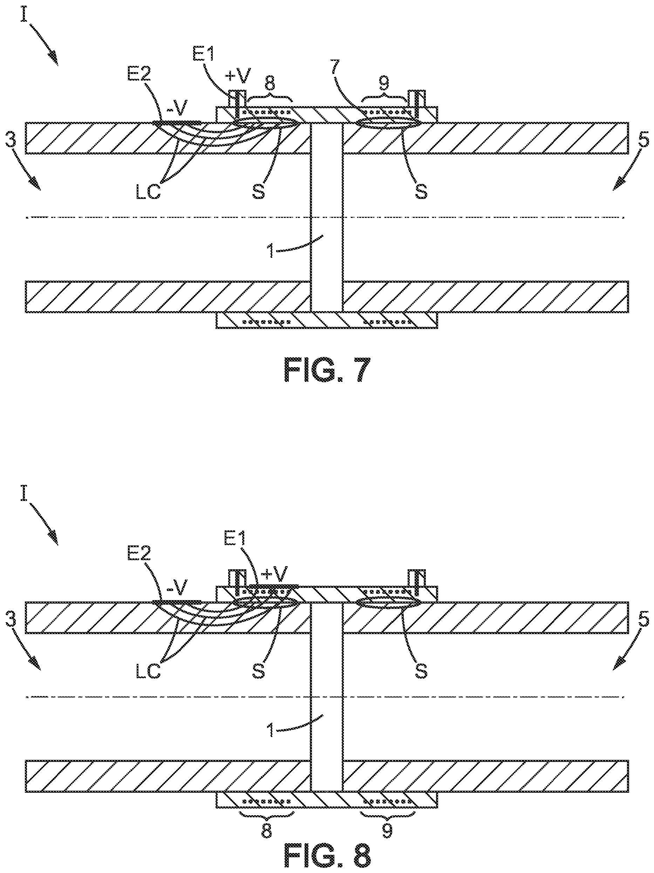

Les figures 7 et 8 illustrent un autre mode de réalisation d'une installation de conduits en PEHD équipés de bobinages de fils électriques.

L'installation I comprend un premier conduit 3 et un second conduit 5 en PEHD. Les conduits 3 et 5 sont joints par leurs extrémités dans une zone de jonction 1 et présentent une forme générale de cylindres coaxiaux.

L'installation I comprend un manchon 7 en PEHD, qui enveloppe les conduits 3 et 5 dans la zone de jonction 1.

Deux bobinages de spire de fils électriques 8, 9, sont enroulés sur le manchon 7, de part et d'autre de la zone de jonction 1 entre les conduits 3 et 5. Le manchon 7 est soudé aux conduits 3 et 5, par exemple par effet du chauffage des bobinages alimentés par une source électrique.

Le procédé de détection de défaut s'applique ici à la détection du défaut de la soudure S entre le manchon 7 et le conduit 3 ou le conduit 5.

Selon une première variante illustrée à la figure 7, l'électrode E1 est constituée par le bobinage 8, l'électrode E2 étant posée sur le conduit 3. Les lignes de champ LC passent à travers la soudure S.

Dans cette variante, la première surface est le manchon 7 tandis que la deuxième surface est la surface de pourtour 2 du conduit 3.

Le procédé permet de détecter un défaut dans la soudure S entre le manchon 7 et le conduit 3. Un défaut dans la soudure S entre le manchon 7 et le conduit 5 peut également être détecté si l'électrode E2 est posée sur le conduit 5.

Selon une deuxième variante illustrée à la figure 8, l'électrode E1 est posée sur le manchon 7 près du bobinage 8 (en étant distincte du bobinage 8), l'électrode E2 étant disposée sur le conduit 3, comme sur la figure 8. Les lignes de champ LC traversent la soudure S.

Le procédé permet de détecter un défaut dans la soudure S entre le manchon 7 et le conduit 3. Un défaut dans la soudure S entre le manchon 7 et le conduit 5 peut également être détecté si l'électrode E1 est posée sur le manchon 7 près du bobinage 9 et si l'électrode E2 est posée sur le conduit 5.

Les bobinages 8, 9, sont illustrés comme étant distincts mais ils peuvent bien entendu faire partie d'un bobinage unique.

Une telle installation I se trouve par exemple dans un réseau de gaz naturel ou d'eau.

Utilisation

L'invention a également pour objet une utilisation de la sonde capacitive précédemment décrite pour la détection de défaut dans une soudure faisant jonction entre une première surface d'un matériau à base de polyéthylène (PE), avantageusement en polyéthylène haute densité et une deuxième surface d'un matériau à base de polyéthylène (PE), avantageusement en polyéthylène haute densité.

Avantageusement, la première surface est coaxiale et adjacente de la deuxième surface. C'est le cas dans le mode de réalisation des figures 1 à 6.

Alternativement, première surface est coaxiale et emmanche en partie au moins la deuxième surface. C'est le cas dans le mode de réalisation des figures 7 et 8.

**Revendications**

1. Procédé de détection d'un défaut dans une soudure faisant jonction entre une première surface d'un matériau à base de polyéthylène (PE) et une deuxième surface d'un matériau à base de polyéthylène (PE), ledit procédé comprenant les étapes :

   - positionnement d'une première électrode (E1) et d'une deuxième électrode (E2) à proximité de la soudure (S), de sorte qu'au moins une ligne de champ électrique traverse ladite soudure (S) quand une différence de potentiel est appliquée entre lesdites première et deuxième électrodes (E1, E2),
   - mesure d'une capacité électrique (Cmes) à une fréquence de mesure entre ladite première électrode et ladite deuxième électrode, la fréquence de mesure étant supérieure à 65 MHz et inférieure à 1 GHz pour comparaison de la capacité électrique mesurée (Cmes) à une valeur de référence (Cref).

2. Procédé selon la revendication 1, dans lequel la fréquence de mesure est mesurée entre 100MHz et 300MHz.

3. Procédé selon la revendication 2, comprenant une étape de détection d'un défaut dans la soudure si, lors de l'étape de comparaison, la capacité électrique mesurée est différente d'au moins 1,5% de la valeur de référence (Cref).

4. Procédé selon la revendication 3, dans lequel la valeur de référence (Cref) est de l'ordre de 6pF.

5. Procédé selon l'une des revendications précédentes, dans lequel la première surface (2) appartient à un corps (3) présentant une épaisseur (e) et la deuxième surface (4) appartient à un corps (5) présentant une épaisseur (e) du même ordre de grandeur que l'épaisseur du corps (3) de la première surface (2), et dans lequel la distance prédéfinie entre la première électrode et la deuxième électrode est comprise dans une plage de valeur entre le cinquième de la valeur de ladite épaisseur (e) et la valeur de ladite épaisseur (e).

6. Utilisation d'une sonde capacitive pour la détection de défaut dans une soudure faisant jonction entre une première surface d'un matériau à base de polyéthylène (PE) et une deuxième surface d'un matériau à base de polyéthylène (PE), la sonde comprenant une première électrode et une deuxième électrode formant condensateur présentant une capacité électrique apte à dépendre de la présence dudit défaut dans la soudure, ladite sonde comprenant un dispositif de mesure mesurant, lors de l'utilisation de ladite sonde, une capacité électrique dans une plage de fréquence de mesure entre 65 MHz et 1 GHz.

7. Utilisation selon la revendication 6, dans laquelle la première surface est coaxiale et adjacente de la deuxième surface.

8. Utilisation selon la revendication 6, dans laquelle la première surface est coaxiale et emmanche en partie au moins la deuxième surface.

**Patentansprüche**

1. Verfahren zur Erkennung eines Fehlers in einer Schweißnaht, die eine Verbindung zwischen einer ersten Oberfläche eines Materials auf Basis von Polyethylen (PE) und einer zweiten Oberfläche eines Materials auf Basis von Polyethylen (PE) herstellt, das Verfahren umfassend die Schritte:

   - Positionieren einer ersten Elektrode (E1) und einer zweiten Elektrode (E2) in der Nähe der Schweißnaht (S), so dass mindestens eine elektrische Feldlinie durch die Schweißnaht (S) verläuft, wenn eine Potentialdifferenz zwischen den ersten und zweiten Elektroden (E1, E2) angelegt wird,
   - Messen einer elektrischen Kapazität (Cmes) mit einer Messfrequenz zwischen der ersten Elektrode und der zweiten Elektrode, wobei die Messfrequenz größer als 65 MHz und kleiner als 1 GHz ist zum Vergleichen der gemessenen elektrischen Kapazität (Cmes) mit einem Referenzwert (Cref).

2. Verfahren nach Anspruch 1, bei dem die Messfrequenz zwischen 100MHz und 300MHz gemessen wird.

3. Verfahren nach Anspruch 2, umfassend einen Schritt zum Erkennen eines Fehlers in der Schweißnaht, wenn sich die gemessene Kapazität der Schweißnaht während des Vergleichsschrittes um mindestens 1,5 % vom Referenzwert (Cref) unterscheidet.

4. Verfahren nach Anspruch 3, bei dem der Referenzwert (Cref) in der Größenordnung von 6pF liegt.

5. Verfahren nach einem der vorstehenden Ansprüche, bei dem die erste Fläche (2) zu einem Körper (3) gehört, der eine Dicke (e) aufweist, und die zweite Fläche (4) zu einem Körper (5) gehört, der eine Dicke (e) der gleichen Größenordnung wie die Dicke des Körpers (3) der ersten Fläche (2) aufweist, und bei dem der vordefinierte Abstand zwischen der ersten Elektrode und der zweiten Elektrode in einem Wertebereich zwischen einem Fünftel des Wertes der Dicke (e) und dem Wert der Dicke (e) liegt.

6. Verwendung einer kapazitiven Sonde zum Erkennen eines Fehlers in einer Schweißnaht, die eine erste Oberfläche eines Materials auf der Basis von Polyethylen (PE) und eine zweite Oberfläche eines Materials auf Basis von Polyethylen (PE) verbindet, wobei die Sonde eine erste Elektrode und eine zweite Elektrode umfasst, welche einen Kondensator bilden, der eine elektrische Kapazität aufweist, die in der Lage ist, von der Anwesenheit des Fehlers in der Schweißnaht abzuhängen, wobei die Sonde eine Messvorrichtung umfasst, die bei Verwendung der Sonde eine elektrische Kapazität in einem Messfrequenzbereich zwischen 65 MHz und 1 GHz misst.

7. Verwendung nach Anspruch 6, wobei die erste Oberfläche zur zweiten Oberfläche koaxial ist und an diese angrenzt.

8. Verwendung nach Anspruch 6, wobei die erste Oberfläche zur zweiten Oberfläche koaxial ist und diese zumindest teilweise umhüllt.

**Claims**

1. A method of detecting a defect in a weld joining a first surface of a polyethylene (PE)-based material and a second surface of a polyethylene (PE)-based material, said method comprising the steps of:

   - positioning a first electrode (E1) and a second electrode (E2) in proximity to the weld (S), so that at least one electric field line passes through said weld (S) when a potential difference is applied between said first and second electrodes (E1, E2),
   - measuring an electrical capacitance (Cmes) at a measurement frequency between said first electrode and said second electrode, the measurement frequency being higher than 65 MHz and lower than 1 GHz in order to compare the measured electrical capacitance (Cmes) to a reference value (Cref).

2. The method according to claim 1, in which the measurement frequency is measured between 100 MHz and 300 MHz.

3. The method according to claim 2, comprising a step of detecting a defect in the weld if, during the comparison step, the measured electrical capacitance is different by at least 1.5% from the reference value (Cref).

4. The method according to claim 3, in which the reference value (Cref) is on the order of 6pF.

5. The method according to claim 1, in which the first surface (2) belongs to a body (3) presenting one thickness (e) and the second surface (4) belongs to a body (5) presenting a thickness (e) of the same order of magnitude as the thickness of the body (3) of the first surface (2), and in which the predefined distance between the first electrode and the second electrode is within a range of values between a fifth of the value of said thickness (e) and the value of said thickness (e).

6. Use of a capacitive probe for detecting a defect in a weld joining a first surface of a polyethylene (PE)-based material and a second surface of a polyethylene (PE)-based material, the probe comprising a first electrode and a second electrode forming a capacitor presenting an electrical capacitance capable of depending on the presence of said defect in said weld, said probe comprising a measuring device measuring, during use of said probe, an electrical capacitance within a measurement frequency of between 65 MHz and 1 GHz.

7. The use according to claim 6, in which the first surface is coaxial and adjacent to the second surface.

8. The use according to claim 6, in which the first surface is coaxial and at least partly fitted to the second surface.

**FIG. 1**

**FIG. 2**

FIG. 3

FIG. 4

**FIG. 5**

**FIG. 6**

FIG. 7

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7839282 B1

**Littérature non-brevet citée dans la description**

- **MATHUR M et al.** A probe for in situ, remote, détection of defects in buried plastic natural gas pipelines. *Review of Scientific Instruments,* 18 Décembre 2007, vol. 78 (12